# EUROPEAN PATENT SPECIFICATION

(11) **EP 0 262 125 B1**
(45) Date of publication and mention of the grant of the patent: **27.03.1996**
(21) Application number: 86902696.3
(22) Date of filing: 08.04.1986
(51) Int. Cl.: A61K 31/70

(54) **AICA riboside for the prophylactic treatment of diseases with restricted blood flow.**
AICA riboside zur prophylaktischen Behandlung von Krankheiten mit einer verminderten Durchblutung.
AICA riboside pour le traitement prophylactique de maladies associees à une circulation reduite.

(30) Priority: 27.03.1986 US 845627
(43) Date of publication of application: 06.04.1988
(60) Divisional application: 94107553.3
(73) Proprietor: GENSIA, INC., San Diego, CA 92121 (US)
(72) Inventor: GRUBER, Harry Edward, San Diego CA 92130 (US)
(74) Representative: Lehn, Werner, Dipl.-Ing.
(86) International application number: US8600736
(87) International publication number: WO8705807

(56) References cited:
- JP-A- 5 334 796
- JP-A-59 167 515
- US-A- 29 835
- US-A- 3 888 843
- US-A- 3 923 785
- US-A- 4 163 839
- US-A- 4 211 771
- US-A- 4 320 134
- US-A- 4 332 806
- US-A- 4 432 990
- US-A- 4 575 498
- Research, volume 51, No. 1, issued 1982, June. (J.L.SWAIN), pages 102-105
- The Journal of Biological Chemistry, Volume 25, No. 17. issued 1982, September, (R.L. SABINA), pages 10178-10183

## Description

### FIELD OF THE INVENTION

This invention relates to the use of AICA riboside for the preparation of a medicament for the prophylactic treatment of diseases associated with undesired restricted blood flow, i.e. diseases that respond beneficially to increases in extracellular levels of adenosine.

Adenosine belongs to the class of biochemicals termed purine nucleosides and is a key biochemical cell regulatory molecule, as described by Fox and Kelly in the Annual Reviews of Biochemistry, Vol. 47, p. 635, 1978, that interacts with a wide variety of cell types, and is responsible for a myriad of biological effects. For instance, adenosine is a potent vasodilator, inhibitor of immune cell function, activator of mast cell degranulation, an inhibitor of granulocyte activation, and a putative inhibitory neurotransmitter. Considering adenosine's broad spectrum of biological activity, considerable effort has been, and continues to be aimed at establishing practical therapeutic uses for the molecule, and its analogs.

Since adenosine or similar purine nucleosides are thought to act at the level of the cell plasma membrane, by binding to receptors anchored in the membrane, past work has focused on increasing the extracellular levels of the molecules. Unfortunately, adenosine or its analogs are toxic at concentrations that have to be administered to a patient to maintain an efficacious extracellular therapeutic level, thus the administration of adenosine alone is of little therapeutic use. Consequently other ways of maintaining high local extracellular levels of adenosine have been sought. The three most used are: a) interference with the uptake of adenosine with reagents that specifically block adenosine transport as described by Paterson et al., in the Annals of the New York Academy of Sciences, Vol. 255, p. 402, 1975; b) prevention of the metabolism of adenosine which, in turn, makes cellular adenosine available for extracellular transport and use outside the cell as described by Carson and Seegmiller in The Journal of Clinical Investigation, Vol. 57, p. 274, 1976; and c) the use of analogs of adenosine constructed to bind to adenosine cell plasma membrane receptors with lower dissociation constants than adenosine.

There exists a large repertoire of chemicals that inhibit the cellular uptake of adenosine. Some do so specifically, and are essentially competitive inhibitors of adenosine uptake, and others inhibit nonspecifically. Theophylline appears to be a competitive inhibitor, while dipyridamole, and a variety of other chemicals, including colchicine, phenethyalcohol and papaverine inhibit uptake nonspecifically.

Methods of increasing the extracellular levels of adenosine by altering adenosine metabolism are primarily founded on the use of chemicals that inhibit enzymatic degradation of adenosine. Most of this work has focused on identifying inhibitors of adenosine deaminase which converts adenosine to inosine. Adenosine deaminase is inhibited by coformycin, 2'-deoxyco-formycin or erythro 9-(2-hydroxy-3-nonyl) adenine hydrochloride.

A number of adenosine analogs have been generated that exhibit structural modifications to the purine ring, alterations in substituent groups attached to the purine ring, and modifications to, or alterations in the site of attachment of the carbohydrate moiety. Halogenated adenosine derivatives appear to be most promising and, as described by Wolff et al. in the Journal of Biological Chemistry, Vol. 252, p. 681, 1977, exert biological effects similar to those caused by adenosine.

Although all three techniques discussed above have advantages over the use of adenosine alone, they have several disadvantages, the major ones being that they rely on chemicals that have adverse therapeutic side effects primarily due to the fact that they must be administered in doses that are toxic, and they affect nonselectively most cell types. As described in Purine Metabolism in Man, (eds. De Baryn, Simmonds and Muller), Plenum Press, New York, 1984, most cells in the body carry receptors for adenosine. Consequently the use of techniques that increase adenosine levels generally throughout the body cause dramatic changes in normal cellular physiology in addition to controlling diseases that benefit from adenosine treatment.

Thus, it will be appreciated from the foregoing discussion that a technique that would increase extracellular levels of adenosine or adenosine analogs without complex side effects, and which would permit increased adenosine levels to be selectively targeted to cells that would benefit most from them, would be of considerable therapeutic use.

### SUMMARY OF THE INVENTION

A novel method is described for alleviating disease based on increasing the extracellular concentration of adenosine, or adenosine analogs utilizing 5-amino-1-(β-D-ribofuranosyl) imidazole-4-carboxamide (AICA riboside). This compound is administered to a patient and is taken up and converted to mono and sometimes triphosphate by cells. Adenosine or inosine are generated from adenosine triphosphate in the course of rapid cellular energy utilization such as during seizure activity or during decreased blood flow by a series or intracellular biochemical reactions. The production of inosine is much greater than that of adenosine (approximately 100 to 1). The compounds subsequently diffuse out of the cell and consequently are present in the immediate extracellular environment at high levels. Nucleosides such as AICA riboside enhance the production of adenosine. The increase in adenosine release is at least partly due to a reduction in 5'nucleotidase activity. It is apparent from the diagram below that with reduced 5'nucleotidase activity IMP is not cleaved as rapidly to inosine and more nucleotide is converted to AMP which builds up and is converted to adenosine. The K_{M} for AMP is approximately 10 fold higher than for IMP. Since adenosine levels are not altered significantly throughout the patient, and alterations only occur in the areas of rapid AMP use, the method of treatment does not cause generalized adenosine release. 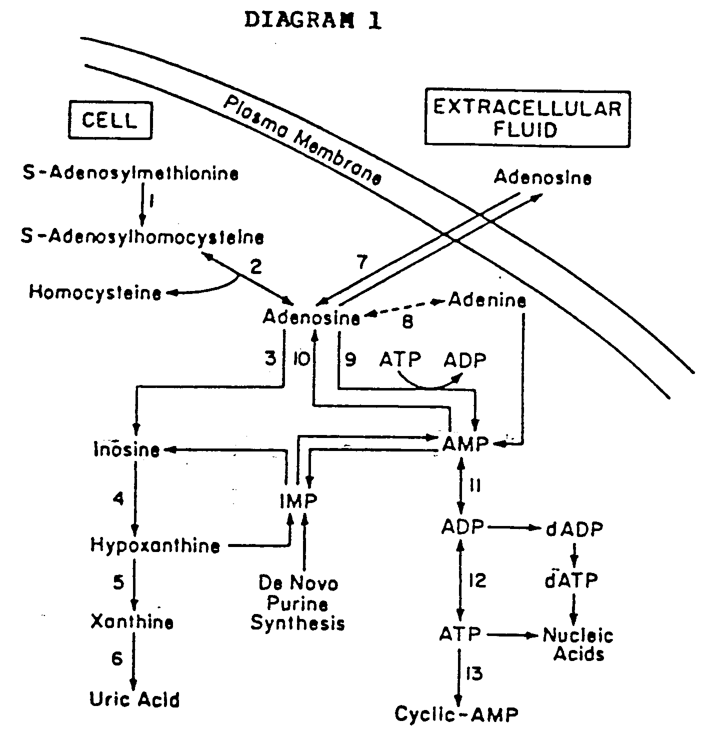
Diagram 1. Metabolism of adenosine. This diagram illustrates the pathways by which adenosine is formed and degraded within cells. Adenosine may also be transported into cells or released from cells. The metabolism of 2'-deoxyadenosine may utilize some of these pathways: 1, S-adenosylmethionine methyltransferases; 2, S-adenosylhomocysteine hydrolase; 3, adenoside deaminase; 4, purine nucleoside phosphorylase; 5 & 6, xanthine oxidase; 7, transport mechanisms; 8 adenosine phosphorylase (not established); 9, adenosine kinase; 10 5'nucleotidase and non-specific phosphatase; 11, adenylate kinase; 12, nucleoside diphosphokinase; 13, adenylate cyclase.

It is therefore apparent that AICA riboside affords a medically beneficial means of establishing a high local extracellular concentration of adenosine without the toxicity, or nonspecific uptake problems associated with other techniques that regulate extracellular adenosine levels.

### BRIEF DESCRIPTION OF THE DRAWINGS

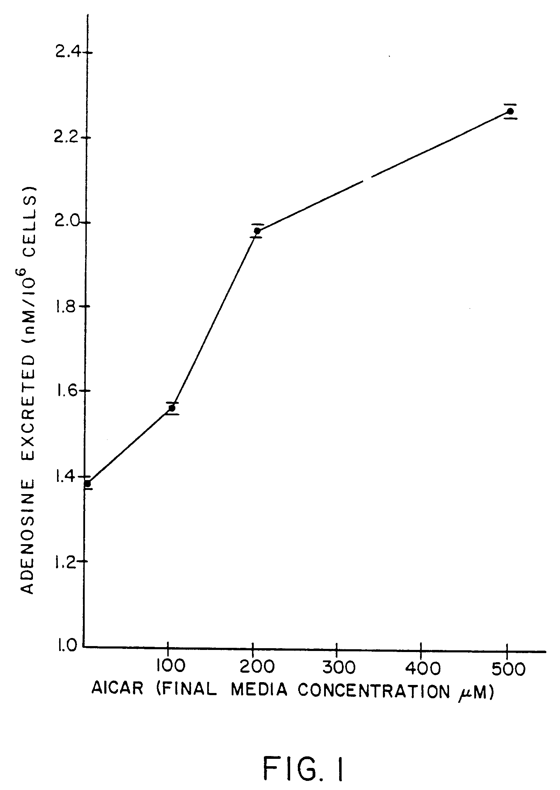

Figure 1. In vitro effect of 5-amino-1-(β-D-ribofuranosyl) imidazole-4-carboxamide on adenosine excretion by lymphocytes.

Figure 2. In vivo effect of 5-amino-(1-β-D-ribofuranosyl) imidazole-4-carboxamide (AICA riboside) on regional myocardial blood flow during coronary artery occlusion in dogs. Regional myocardial blood flow was measured using radiolabelled microspheres infused into the left atrium at 5 (open) and 60 (hatched) minutes of occlusion. The means plus the standard deviations are graphed.

Figure 3. The effect of AICA riboside treatment on coronary venous adenosine concentrations. Coronary venous blood was collected into equal volumes of chilled 2N perchloric acid at various times before and after coronary artery occlusion. Supernatant from these extracts were neutralized with alamine and freon and evaluated by high performance liquid chromotography. The mean adenosine concentrations +/- standard deviations for the 5 saline treated (●) and 6 AICA riboside treated (□) dogs are graphed.

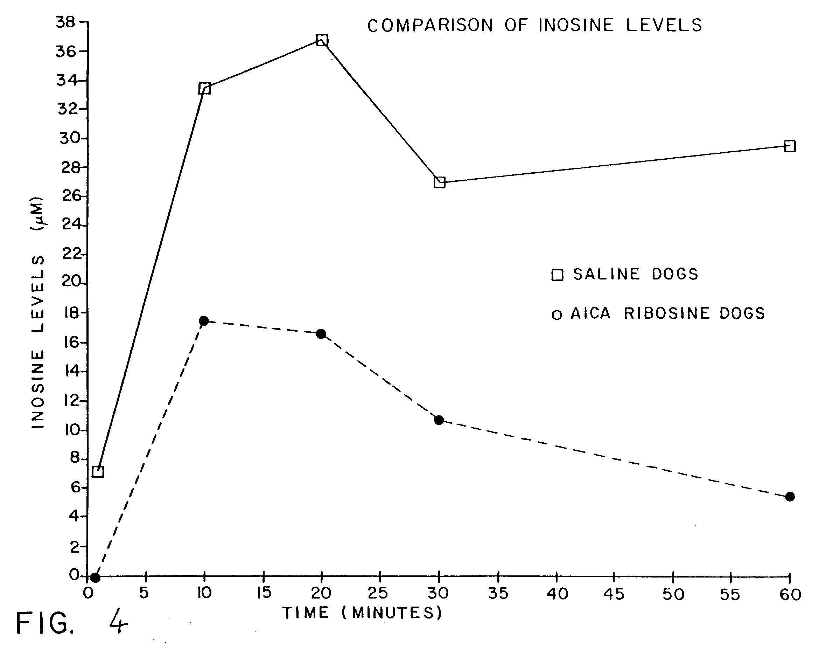

Figure 4. Comparison of the effects of of AICA riboside in saline (○) and saline (□) on inosine levels.

### DETAILED DESCRIPTION OF THE INVENTION

The effect of AICA riboside on agenosine transport can be demonstrated either in vitro or in vivo where the effective concentration of either molecule is 10µM-100µM. When delivered to cells in vitro AICA riboside can be dissolved in aqueous solution and added directly to the culture media. To deliver AICA riboside to patients, it is anticipated that the same may be administered orally since it is not readily degraded by enzymes in the body, or by exposure to low pH presence in the stomach. There is no a priori reason for believing that the drug cannot also be administered intravenously, or by direct intramuscular injection, topically, or by inhalation.

Upon contact with activated energy-requiring target cells, AICA riboside is transported intracellularly by a plasma-membrane bound facilitated diffusion transport system where it can be phosphorylated by adenosine kinase to yield nucleotide monophosphate. The latter chemical can be converted to the triphosphate, and is an intermediate in the de novo synthesis of purine nucleotides.

In order to regulate the amount of adenosine, and hence the effectiveness of AICA riboside treatment, the degree of increased extracellular transport is monitored by isolating the aqueous fluid that bathes the cells, and determining the amount present in the media by chromatographic techniques as described by Matsumoto et al. in The Journal of Biological Chemistry, Vol. 254, p. 8956, 1979. In the tissue culture experiments, one millimolar deoxycoformycin was added to the aqueous fluid to prevent destruction of adenosine by adenosine deaminase. In addition, the amount of adenosine remaining in the cells is analyzed by high pressure liquid column chromatography after isolation from the cells as described by Matsumoto et al., in The Journal of Biological Chemistry, Vol. 254, p. 8956, 1979.

Since the amount of extracellular adenosine can be regulated by increasing or decreasing the concentration of the AICA riboside present in the cellular growth environment, if the adenosine level is too low to be medically beneficial, it can be increased by increasing the amount of AICA riboside administered. Similarly, if the amount of adenosine is too great, it can be reduced by reducing the amount of AICA riboside.

It is anticipated that AICA riboside treatment will benefit patients suffering from a variety of illnesses.

It should be possible to use AICA riboside to treat diseases that arise from, or are aggravated by, insufficient blood flow through a particular organ. For example, angina pectoris, transient ischemic attacks, or migraine headaches can be treated by administering AICA riboside. This is expected since adenosine is known to be a potent vasodilator acting by reducing vascular smooth muscle contraction.

In addition to acting as a vasodilator, AICA riboside increases collateral blood flow by a second mechanism. Often granulocytes stick to microvasculature in the region of restricted blood flow. AICA riboside causes granulocytes to dislodge, and in doing so help to reestablish normal blood flow. Thus, the uptake of AICA riboside by smooth muscle cells followed by subsequent release of adenosine should cause vasodilation and/or removal of granulocytes.

Another disease associated with restricted blood flow is myocardial arrhythmia. Although restricted blood flow initiates the onset of arrhythmia, the precise cause is unknown. However, it is known that lipid peroxidation by oxygen radicals is arrhythmogenic. Since the latter are secreted by granulocytes, AICA riboside inhibition of granulocyte activation can be expected to control arrhythmia. In addition, granulocytes are in higher concentration in areas of arteriosclerosis. Suppression of their activation might reduce the release of other mediators of arrhythmias.

### EXAMPLE I

### In vivo effect of AICA riboside on adenosine levels and increased blood flow in dogs

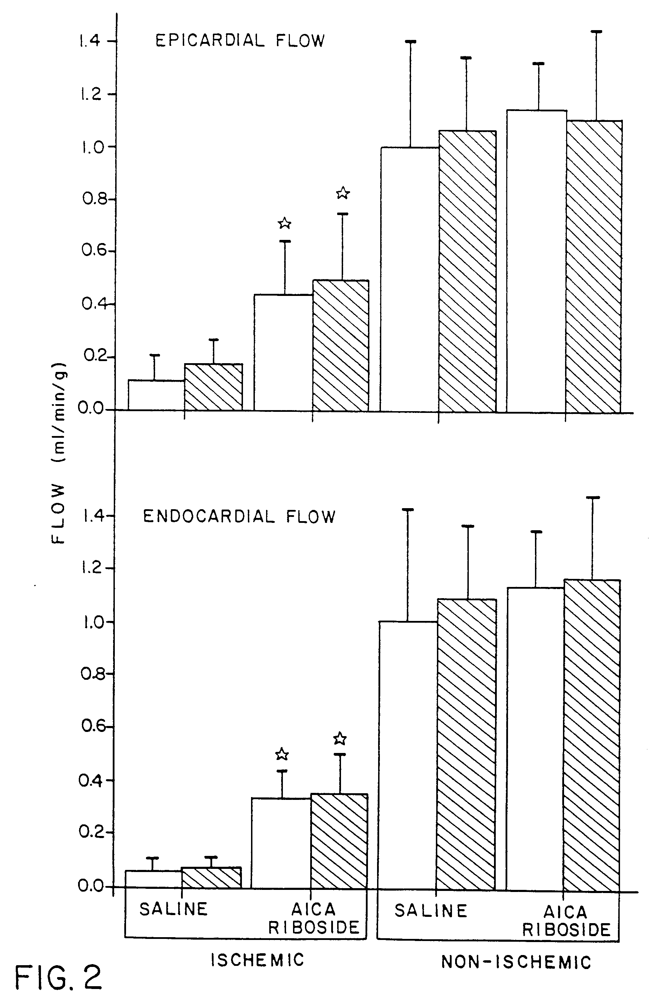

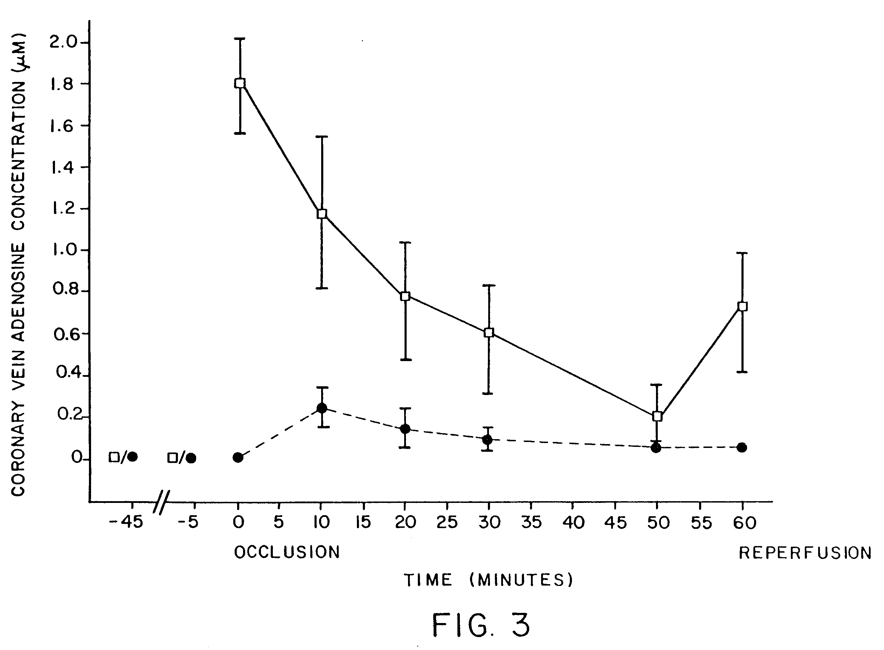

Figures 2 and 3 show the results of a series of experiments carried out to demonstrate the effects of AICA ribosides on adenosine levels in blood, and to correlate the increase in adenosine with increased blood flow. Thirteen mongrel dogs were anesthetized with phenobarbital. The anterior coronary vein was cannulated and a blood sample was collected into 2N perchloric acid. Saline or 100mM AICA riboside in saline was randomly selected for infusion into the femoral vein for 45 minutes prior to coronary artery occlusion at a rate of 1 ml/min. Coronary venous blood was collected and assayed for adenosine as described in Example 1 5 minutes prior to occlusion, and after 1, 10, 20, 30 and 50 minutes of occlusion of the left anterior descending coronary artery as well as 1 minute after reperfusion. Regional myocardial blood flow was measured with 15 µm radiolabelled spheres infused into the left atrium at 5 and 60 minutes during the ischemic period as described by Heymann et al. in Prog. C.V. Dis. 20, 55 (1977). The electrocardiogram and arterial pressure were monitored throughout the period of ischemia. Six AICA riboside treated and five saline treated dogs survived the procedure. Two of the surviving saline treated animals fibrillated. The concentration of AICA riboside in AICA riboside treated dogs immediately before occlusion was 57.4 +/- 40.2µM. The range was 4.4 to 100µM.

Figure 2 shows that adenosine levels are dramatically increased upon AICA riboside perfusion, while Figure 3 shows that regional myocardial blood flow to the ischemic myocardium was significantly greater in AICA riboside than in saline treated animals. A similar degree of difference in flow was seen in endocardium and epicardium, and there were no changes between 5 and 60 minutes of ischemia. AICA riboside did not alter flow to normal myocardium, as the non-ischemic tissue flow rates are remarkably similar between the two groups. Systemic arterial pressure and heart rate at 5 and 60 minutes showed no significant differences between the two groups of dogs. Arterial blood gas-content and systemic venous granulocyte counts were not significantly different between the two groups.

We conclude that AICA riboside enhances collateral coronary flow to ischemic myocardium possibly by augmenting adenosine release and thus vasodilating and/or inhibiting granulocyte capillary plugging.

### EXAMPLE II

### Use of 5-amino-(1-β-D-ribofuranosyl)-imidazole-4-carboxamide to treat angina.

To show the efficaciousness of AICA riboside for treating heart disease, a dog model of angina was developed. This consists of partially obstructing by placing a ring about the proximate portion of the coronary arteries feeding the dog myocardium. The ring slowly absorbs fluid and further constricts the artery over several weeks. Consequently when the dogs are exercised, they display the classical electrocardiogram and wall tension changes associated with angina. These dogs were treated with drugs known to relieve angina in humans, such as Nifedipine, and were shown to be of no help. However, when the same dogs were subsequently treated with AICA riboside, there was a dramatic, approximately 30%, increase in blood flow through the coronary arteries as measured by doppler readings. Moreover, there was also at least a two-fold increase in myocardium contractility in the ischemic region as measured by wall thickness.

### EXAMPLE III

### Effect of AICA riboside treatment on arrhythmias

One consequence of myocardial ischemia is arrhythmia and the frequency of arrhythmias is related to the degree of blood flow. Therefore, the effect of AICA riboside treatment on arrhythmias was determined. Electrocardiograms recorded during ischemia were analyzed for the number of premature ventricular depolarizations (PVD) and ventricular tachycardia (VTAC) episodes. Table 1 shows that the saline treated dogs had 112.2 PVD and 18.2 episodes of VTAC during ischemia as compared to 37.8 PVD and 4.7 episodes of VTAC for the AICA treated animals (p ¼ 0.01). The one AICA riboside treated dog (#3) with frequent arrhythmias had much lower collateral blood flow rates and adenosine concentrations (but an AICA riboside blood concentration of 27.2 M) compared to the other AICA riboside treated dogs.

**TABLE 1**

| TREATMENT GROUP | ARRYTHIMIAS (EPISODES/H) | |
|---|---|---|
| SALINE | PVC | VTAC |
| 1 | 101 | 10 |
| 2 | 144 | 23 |
| 3 | 32 | 44 |
| 4 | 57 | 8 |
| 5 | 27 | 6 |
| AVERAGE | 118.2 | 18.2 |

| AICA RIBOSIDE | PVC | VTAC |
|---|---|---|
| 1 | 12 | 1 |
| 2 | 10 | 0 |
| 3 | 182 | 27 |
| 4 | 4 | 0 |
| 5 | 13 | 0 |
| 6 | 6 | 0 |
| AVERAGE | 37.8 | 4.7 |

Prior to ischemia none of the dogs had measurable venous adenosine (< 0.01µM) before and during AICA riboside or saline infusion. The saline treated animals had a peak adenosine level at 10 minutes after occlusion (0.22 +/-0.08µM) which fell to an undetectable level by 60 minutes. In contrast, the AICA riboside treated animals had a peak adenosine level at 1 minute of ischemia (1.79 +/- 0.35µM) which remained elevated at 60 minutes (0.18 +/- 0.15). Reperfusion resulted in no detectable adenosine washout in saline treated animals but a significant rise in the AICA treated animals.

### EXAMPLE IV

### Effect of AICA riboside treatment on inosine levels

That the increase in the levels of adenosine is due, at least in part, to a reduction in inosine levels was shown by treating dogs as described in Example IV with AICA riboside followed by analysis of venous blood for inosine levels. Figure 6 shows a more than 2 fold decrease in inosine levels over a 60 minute assay period.

### EXAMPLE V

The effect of AICA riboside treatment on coronary infarct size was determined in rats by inducing restricted blood flow by tying off the coronary artery and subsequently dosing the animals with either AICA riboside in saline, or saline or alone. Further, the animals were continuously exposed by infusion of either AICA riboside or a saline using osmotic mini-pumps well-known to those in the art. After three weeks the rats were sacrificed and infarct size quantitated by planinarizing stained sections of fixed hearts. The results showed that in AICA riboside treated rats there is a reduction of infarct size of approximately 24% compared to saline-treated controls.

## Claims

1. The use of AICA riboside for the preparation of a medicament for the prophylactic treatment of diseases associated with undesired restricted blood flow.

2. The use according to claim 1, where said AICA riboside is formulated so that upon administration the blood concentration of AICA riboside is in the range of 4.4 to 100 µM.

3. The use according to claim 1 to locally increase adenosine concentrations in areas of rapid AMP use.

4. The use according to any of the preceding claims, wherein said diseases associated with restricted blood flow are diseases of the heart.

5. The use according to any of the preceding claims, wherein said heart disease is angina.

6. The use of AICA riboside for the preparation of a medicament for use as a vasodilator.

7. The use of AICA riboside according to any of the preceding claims as a local vasodilator.

8. The use according to any of the preceding claims against myocardial arrhythmia.

9. The use according to any of the preceding claims 1 to 3 against infarction of the heart.

10. The use according to claim 1 against transient ischemic attacks.

11. The use according to claim 1 against myocardial ischemia.

12. The use according to claim 1 against coronary artery occlusion.

13. The use according to any of the preceding claims, wherein AICA riboside is administered orally, intramuscularly, topically or by inhalation.

14. The use according to any of the preceding claims, wherein AICA risobise is administered intraveneously.

## Patentansprüche

1. Verwendung von AICA-Ribosid zur Herstellung eines Arzneimittels für die prophylaktische Behandlung von Krankheiten, die mit unerwünschtem verminderten Blutfluß einhergehen.

2. Verwendung nach Anspruch 1, worin das AICA-Ribosid so formuliert wird, daß nach Verabreichung die Blutkonzentration von AICA-Ribosid im Bereich von 4,4 bis 100 µM liegt.

3. Verwendung nach Anspruch 1 zur lokalen Erhöhung der Adenosinkonzentrationen in Gebieten von rascher AMP-Verwertung.

4. Verwendung nach einem der vorhergehenden Ansprüche, worin die mit vermindertem Blutdruck einhergehen Erkrankungen solche des Herzens sind.

5. Verwendung nach einem der vorhergehenden Ansprüche, worin die Herzerkrankung Angina ist.

6. Verwendung von AICA-Ribosid zur Herstellung eines Arzneimittels zur Verwendung als Vasodilator.

7. Verwendung von AICA-Ribosid nach einem der vorhergehenden Ansprüche als lokaler Vasodilator.

8. Verwendung nach einem der vorhergehenden Ansprüche gegen myokardiale Arrhythmie.

9. Verwendung nach einem der vorhergehenden Ansprüche 1 bis 3 gegen Herzinfarkt.

10. Verwendung nach Anspruch 1 gegen vorübergehende ischämische Anfälle.

11. Verwendung nach Anspruch 1 gegen myokardiale Ischämie.

12. Verwendung nach Anspruch 1 gegen koronaren Arterienverschluß.

13. Verwendung nach einem der vorhergehenden Ansprüche, worin AICA-Ribosid oral, intramuskulär, topisch oder durch Inhalation verabreicht wird.

14. Verwendung nach einem der vorhergehenden Ansprüche, worin AICA-Ribosid intravenös verabreicht wird.

## Revendications

1. Utilisation du AICA riboside pour la préparation d'un médicament pour le traitement prophylactique de maladies associées à une circulation sanguine réduite non désirée.

2. Utilisation selon la revendication 1, dans laquelle ledit AICA riboside est formulé de manière que à l'administration la concentration dans le sang du AICA riboside se situe dans la gamme de 4,4 à 100 µM.

3. Utilisation selon la revendication 1 pour augmenter localement les concentrations d'adénosine dans des zones d'utilisation rapide de l'AMP.

4. Utilisation selon l'une quelconque des revendications précédentes, dans laquelle lesdites maladies associées à une circulation sanguine réduite sont des maladies cardiaques.

5. Utilisation selon l'une quelconque des revendications précédentes, dans laquelle ladite maladie cardiaque est une angine.

6. Utilisation du AICA riboside pour la préparation d'un médicament pour utilisation comme vasodilatateur.

7. Utilisation du AICA riboside selon l'une quelconque des revendications précédentes comme vasodilatateur local.

8. Utilisation selon l'une quelconque des revendications précédentes contre l'arythmie du myocarde.

9. Utilisation selon l'une quelconque des revendications précédentes 1 à 3, contre l'infarctus cardiaque.

10. Utilisation selon la revendication 1 contre les attaques ischémiques passagères.

11. Utilisation selon la revendication 1 contre l'ischémie du myocarde.

12. Utilisation selon la revendication 1 contre l'occlusion des artères coronaires.

13. Utilisation selon l'une quelconque des revendications précédentes, dans laquelle le AICA riboside est administré oralement, intramusculairement, topiquement ou par inhalation.

14. Utilisation selon l'une quelconque des revendications précédentes, dans laquelle le AICA riboside est administré par voie intraveineuse.
